# EUROPÄISCHE PATENTSCHRIFT

(11) **EP 1 130 980 B1**
(45) Veröffentlichungstag und Bekanntmachung des Hinweises auf die Patenterteilung: **03.03.2004**
(21) Anmeldenummer: 99955588.1
(22) Anmeldetag: 19.11.1999
(51) Int. Cl.: A23L 2/38, A23L 1/302, A23L 1/304, A23L 1/30, A61K 31/51, A61K 31/525, A61K 31/185, A61K 31/70, A61K 31/455, A61K 31/505

(54) **GETRÄNK ZUR STEIGERUNG DER ALKOHOL-ABBAU-KAPAZITÄT**
BEVERAGE FOR INCREASING THE BODY'S CAPACITY TO BREAK DOWN ALCOHOL
BOISSON POUR AUGMENTER LA CAPACITE A DEGRADER L'ALCOOL

(30) Priorität: 19.11.1998 AT 193498
(43) Veröffentlichungstag der Anmeldung: 12.09.2001
(73) Patentinhaber: Fuchs, Norbert, 5580 Unternberg (AT); Wallner, Reinhard, 5580 Tamsweg (AT)
(72) Erfinder: Fuchs, Norbert, 5580 Unternberg (AT); Wallner, Reinhard, 5580 Tamsweg (AT)
(74) Vertreter: Alge, Daniel, Mag. Dr. rer.nat.
(86) Internationale Anmeldenummer: PCT/AT1999/000282
(87) Internationale Veröffentlichungsnummer: WO 2000/030477

(56) Entgegenhaltungen:
- EP-A- 0 583 852
- EP-A- 0 652 012
- WO-A-87/01285
- WO-A-97/02830
- DE-A- 19 720 818
- DATABASE WPI Section Ch, Week 199621 Derwent Publications Ltd., London, GB; Class B05, AN 1996-205436 XP002130552 & JP 08 073350 A (ITOEN KK), 19. März 1996 (1996-03-19)
- DATABASE WPI Derwent Publications Ltd., London, GB; AN 1997-527237 XP002130553 & CN 1 126 588 A (LIHH-I), 17. Juli 1996 (1996-07-17)
- DATABASE WPI Section Ch, Week 199812 Derwent Publications Ltd., London, GB; Class B04, AN 1995-307124 XP002130554 & JP 02 713392 B (HAGIWARA Y), 16. Februar 1998 (1998-02-16)

## Beschreibung

Die Erfindung betrifft die Verwendung einer Erfrischungsgetränk-Zusammensetzung zur Steigerung der Alkohol-Abbau-Kapazität des Körpers enthaltend Fructose.

Die Erfindung betrifft weiters ein Erfrischungsgetränk, einen Sirup sowie eine Trockensubstanz zur Steigerung der Alkohol-Abbau-Kapazität des Körpers.

Die Resorption von Alkohol (Ethanol) erfolgt sowohl vom Magen als auch vom Darm aus. In der Regel ist die Resorption in etwa einer Stunde beendet, in nüchternem Zustand allerdings früher, bei starker Magen/Darm-Füllung kann die Resorption auch verzögert sein. Aufgrund des Öl/Wasser-Verteilungsquotienten von 0,04 erfolgt eine rasche Verteilung des Alkohols im Körperwasser. Wegen dieses raschen Konzentrationsausgleichs gilt der Blutalkoholspiegel als repräsentativ für die Konzentration des Alkohols im Zentralnervensystem (ZNS), dem wesentlichen Wirkungsort.

Etwa 2-3% des resorbierten Alkohols werden über die Lunge ausgeschieden, etwa 1-2% über die Nieren. Die Hauptmenge jedoch wird in der Leber verstoffwechselt. Dabei wird Ethylalkohol von der Alkohol-Dehydrogenase (ADH) zu Acetaldehyd oxidiert. Das Enzym ADH trägt als katalytisches Zentrum Zink und ist NADH-abhängig. Daneben aber werden geringe Anteile des Alkohols auch über das P450-abhängige Enzymsystem der Monooxygenasen ebenfalls zu Acetaldehyd bzw. Essigsäure oxidiert. Geringe Mengen (0,5%) werden direkt glucuronidiert, Spuren an Schwefelsäure gekoppelt und mit dem Harn ausgeschieden. Der durch ADH gebildete Acetaldehyd wird durch das Enzym Aldehyd-Dehydrogenase zu Essigsäure weiter oxidiert. Die so angefallene Essigsäure wird zum Teil im Intermediärstoffwechsel über die Aktivierung von Coenzym A verwertet, zum größtern Teil jedoch wird die Essigsäure im Tricarbonsäurezyklus in CO₂ und H₂O aufgespalten. Ein Gramm Ethanol liefert 7,1 kcal (etwa 30 kJ) und kann somit auch als Teilenergiequelle dienen.

Ein Erfrischungsgetränk zur Senkung des Blutalkoholspiegels ist in der EP 0 205 634 A oder in der DE-C1 4 431 178 beschrieben, das Wasser, Fructose, Ascorbinsäure und Aromastoffe und/oder Zitronensäure und/oder Chinin enthält. Die Alkohol-Abbau beschleunigende Wirkung von Fructose ist seit langem bekannt, der Zusatz von Ascorbinsäure (Vitamin C) wirkt abbaufördernd.

Weiters ist ein Getränk zur Beschleunigung des Abbaus von Alkohol im Körper aus dem Artikel "Alko-Killer" auf S. 44 der Zeitschrift "PRAXIS", Nr. 8-9/98 bekannt. Dieses Getränk enthält neben Fructose und Vitamin C die Coenzyme, NAD+/NADH (Nicotinamid-Adenin-Dinucleotid). NAD+/NADH sind Coenzyme, auf die der Wasserstoff, der bei der Oxidation von Ethanol zu Acetaldehyd durch die Alkohol-Dehydrogenase entsteht, übertragen wird. Die Alkohol-Dehydrogenase ist ein Enzym, das in der Leber 90% des konsumierten Ethanols metabolisiert. Durch den Zusatz der Coenzyme NAD+/NADH in das Getränk steigt durch Konsumieren des Getränks der Gehalt dieser Coenzyme im Körper und damit die Alkoholabbaugeschwindigkeit. Nachteilig ist aber hierbei, dass NAD+ - selbst bei 4°C - zersetzt wird, und NADH wird in sauren und wässrigen Lösungen zu Dehydrogenase-Hemmstoffen zersetzt, was den Alkohol-Abbau in der Leber jedoch hindert. Wird also dieses Getränk längere Zeit gelagert (selbst bei 4°C im Kühlschrank), so verliert es seine Wirkung oder kann gar eine gegenteilige Wirkung entfalten.

Die WO 87/01285 A offenbart eine therapeutische Zusammensetzung zur Behandlung von akuten und/oder chronischen Symptomen, die in Verbindung mit exzessiver Aufnahme von Alkohol auftreten. Diese Zusammensetzung umfasst ein Analgetika sowie Nikotinamid und/oder NAD. Die Zusammensetzung kann gegebenenfalls auch Fructose, wasserlösliche Vitamine, ein Antazidum, einen Elektrolytenersatz, z.B. Kalium, Natrium, Magnesium oder Calcium, Spurenmetalle, z.B. Zink-Ionen, eine Antihistaminkomponente, Alkaloide, Coffein und weitere Zusätze, z.B. Geschmacks- und Süßstoffe, umfassen. Dabei ist es für die Wirkung der Zusammensetzung besonders wichtig, dass die Nikotinamide bzw. das NAD zu zumindest 7 Gew.% der Analgetika zugesetzt werden. Analgetika sind Schmerzmittel, wobei die stärkeren bei Patienten Sucht-Potential entwickeln und die schwächeren Analgetika Nebenwirkungen wie die Reduktion der Magen- und Darmschleimhaut-Produktion hervorrufen. Die Einnahme einer Zusammensetzung mit relativ starken Nebenwirkungen ist für die Behandlung von Symptomen eines übermäßigen Alkoholgenusses nicht günstig und kann bei Personen, die diese Zusammensetzung häufig einnehmen, zu gesundheitlichen Schäden führen.

Die CN 1 090 146 A offenbart ein Gesundheitsgetränk, das unter anderem wasserlösliches Vitamin B, Fructose, Spuren elemente, Vitamin A, Vitamin C, Aminosäuren umfasst und eine Wirkung gegen-Resttoxizität von Alkohol aufweist.

In der KR 9 500 456 B1 wird ein Getränk gegen Wirkungen von Alkohol beschrieben, wobei dieses Getränk unter anderem Fructose zu 1-10% und Vitamin B2 zu 0,05-0,5% sowie ein Filtrat einer Fermentation mit Milchsäurebakterien umfasst. Die Fermentation wird bei bestimmten Bedingungen (30-40°C, pH 9-9.5, 50 bis 100 Stunden) durchgeführt. Die Herstellung dieses Getränks ist relativ aufwendig, was sich in der Regel auch auf den Preis des Getränks auswirkt.

Die JP 61162159 A offenbart ein Getränk zur Beschleunigung der Reduzierung von Alkohol im Blut, wobei das Getränk Fructose, Vitamin C und Chinin oder Chininderivate umfasst.

In der DE 197 20 818 A wird eine Zusammensetzung zur Leistungsförderung bei sportlicher Tätigkeit beschrieben, die verschiedene Elektrolyte, Vitamine und Spurenelemente mit und ohne L-Methionin als Trockenpulver oder in Wasser gelöst umfasst.

In der EP 0 652 012 A wird eine Zusammensetzung beschrieben, die den Durchtritt von Substanzen durch die Blut/Hirn-Schranke erleichtern und fördern soll, wobei diese Zusammensetzung eine Kombination von Zuckern und Aminosäuren ist und für die verschiedensten Behandlungen eingesetzt werden kann. In dieser Schrift ist eine Basiszusammensetzung angegeben. Diese Basiszusammensetzung kann je nach Behandlung verschiedene weitere Zusatzstoffe umfassen: Als Beispiel wird angeführt, dass die Zusammensetzung zur Wiederherstellung der motorischen Funktionen nach überhöhtem Alkoholgenuss bzw. zur Behandlung von Alkoholikern eingesetzt werden kann, wobei der Basiszusammensetzung gemäß Beispiel 1 L-Arginin und/oder Hydroxyprolin sowie Geschmacksstoffe zugesetzt werden. Die Wirkstoffe wie L-Arginin und Hydroxyprolin in dieser Zusammensetzung verbessern die motorischen Disfunktionen nach überhöhtem Alkoholgenuss in der Folge einer erleichterten Blut/Hirn-Passage. Die Wirkstoffe dieser Zusammensetzung wirken somit auf das Zentralnervensystem, und es wird explizit beschrieben, dass die motorischen Funktionen des Patienten wieder hergestellt werden, ohne jedoch eine Wirkung auf den Alkoholgehalt im Blut des Patienten auszuüben.

Die Derwent Publ. XP002130552 & JP 08 073350 A (Itoen KK) betrifft eine Zusammensetzung zur Behandlung der Alzheimerschen Krankheit, Parkinsonschen Krankheit, Senilität und Nervenkrankheiten, wobei die zu verabreichende Zusammensetzung unter anderem Fruchtzucker, Koffein, Tein, Nikotinamide, Inositol und Taurin umfasst.

Die Derwent Publ. XP002130553 & CN 1 126 588 (LIHH-I) betrifft ganz allgemein ein Gesundheitsgetränk umfassend Taurin, Inositol, Vitamin B, Koffein, Sorbitol, Zucker und andere Inhaltsstoffe.

Gemäß der EP 0 583 852 A wird eine Flaschenkürbispulver-Zusammensetzung als Nahrungsmittel oder Getränk beschrieben, die zur Kopierung des eigenwilligen Geruchs und Geschmacks von Flaschenkürbis-Konzentraten dient. Das Ziel dieser Erfindung ist somit vorwiegend lebensmitteltechnischer Natur.

In der WO 97/02830 A wird ein Verfahren zur Prävention von Otitis media in Säuglingen und Kleinkindern beschrieben, wobei die zu verabreichende Zusammensetzung u.a. Fructooligosaccharide, Fructosane, Xylooligosaccharide und Galactooligosaccharide umfasst. Durch die probiotischen Faktoren wird das immunsystem gestärkt, wodurch die Häufigkeit von z.B. Otitis media herabgesetzt wird.

Es ist daher Ziel der vorliegenden Erfindung, eine Verwendung einer Erfrischungsgetränk-Zusammensetzung zur Verfügung zu stellen, wobei die Alkohol-Abbaugeschwindigkeit des Körpers - auch im Vergleich zu Fructose(Vitamin C)-Mischungen - bedeutend erhöht wird, ohne jedoch Enzyme bzw. NADH+/NAD zuzusetzen, so dass die Lebensdauer des Produktes dadurch nicht beschränkt ist. Dabei soll die Zusammensetzung weiters keine Stoffe umfassen, die Nebenwirkungen hervorrufen und die durch häufiges Einnehmen zu gesundheitlichen Schäden führen. Ein weiteres Ziel der vorliegenden Erfindung ist es, ein Erfrischungsgetränk, einen Sirup und eine Trockensubstanz mit diesen Eigenschaften zur Verfügung zu stellen.

Die erfindungsgemäße Verwendung der eingangs angeführten Art ist dadurch gekennzeichnet, dass die Erfrischungsgetränkzusammensetzung neben Fructose eine oder mehrere Komponenten des Vitamin-B-Komplexes sowie Taurin umfasst. Auf diese Weise lässt sich überraschenderweise die Geschwindigkeit des Alkohol-Abbaus im Körper äußerst effektiv, bevorzugterweise um mindestens 45% (siehe Beispiele), steigern. Dabei kann die zugesetzte Fructose in jeder bekannten Form vorliegen, sowie auch phosphoryliert sein. Die erfindungsgemäße Zusammensetzung weist eine hohe Stabilität auf und ist somit über einen längeren Zeitraum hin lagerbar.

Als Vitamin-B-Komplex werden alle wasserlöslichen Vitamine außer Vitamin C definiert. In vielen Fällen sind sie Bestandteile von Coenzymen (siehe unten), die in Redox-Reaktionen aktiv sind und somit direkt oder indirekt die Reaktion der Alkohol-Dehydrogenase fördern. Insbesondere die Aktivierung des Tricarbonsäurezyklus durch die Vitamine des B-Komplexes stellt für die vorliegende Erfindung eine wesentliche Eigenschaft dar, da sich herausgestellt hat, dass sich durch die Aktivierung des Citratzyklus auch die Akloholabbaugeschwindigkeit entscheidend steigern lässt.

Taurin ist die Bezeichnung von 2-Aminoethansulfonsäure und kommt in fast allen Säugetier-Arten vor. Taurin spielt eine wichtige Rolle bei der Entwicklung des zentralen Nervensystems (ZNS) und beeinflusst Transportvorgänge 2-wertiger Metall-Ionen, z.B. als Calcium-, Magnesium- und Zink-Modulator. Taurin wirkt weiter als inhibierender Neurotransmitter oder Neuromodulator. Relativ hohe Konzentrationen von Taurin findet man im ZNS, in der Retina und im Herz. Es wurde gezeigt, dass Taurin-Defizite bei Epilepsie, Mongolismus, Sehschwächen und Herzrhythmusstörungen eine Rolle spielen können. Die Taurin-Ausscheidung wird durch die Nieren gesteuert, wobei Taurin-Mangel zu abnormaler Hirnentwicklung führen kann.

Es konnte ebenfalls überraschenderweise gezeigt werden, dass Taurin den Alkohol-Abbau im Körper unterstützt und beschleunigt. Durch Zusatz von Taurin zu einer Zusammensetzung umfassend weiters Fructose und zumindest eine Vitamin B-Komplex-Komponente wird ein Getränk zur Verfügung gestellt, das außerordentlich effektiv den Alkohol im Körper abbaut. Diese (zumindest) drei Komponenten weisen, wenn sie in einer Zusammensetzung enthalten sind, überraschenderweise einen synergistischen Effekt auf, d.h. dass die Komponenten in einer funktionellen Wechselwirkung zueinander stehen und einen über die Summe ihrer Einzelwirkungen hinausgehenden kombinatorischen Effekt aufweisen. Die Kombination dieser (zumindest) drei Komponenten hat eine außerordentlich starke Alkohol abbauende Wirkung. Dadurch können die Auswirkungen eines exzessiven Alkoholgenusses, z.B. Kopfschmerzen, Herz-Kreislauf-Störungen, Durst, Übelkeit, Zittern, Schwindelgefühl, Müdigkeit, Koordinationsstörungen etc. wesentlich wirksamer und schneller bekämpft werden, als mit den herkömmlichen, bereits bekannten Mitteln.

Diese Getränk-Zusammensetzung kann auch als begleitende Behandlung in besonders extremen Fällen verabreicht werden, etwa bei Alkoholvergiftungen mit Koma, Kreislaufzusammenbruch, Krämpfen, etc..

Eine weitere Anwendung ist die Behandlung von Alkoholikern, die chronische Symptome aufweisen, wie chronischer Elektrolyten- und Flüssigkeitsverlust, Zittern, Müdigkeit, Koordinationsstörungen, körperliche Schwächen sowie mentale Probleme, wie Depressionen, Halluzinationen, etc..

Eine besonders günstige Verwendung ist dadurch gegeben, dass die Zusammensetzung Fructose und Vitamin-B-Komplex-Komponenten in einem Gewichtsverhältnis von 10:1 bis 10.000:1, insbesondere von 100:1 bis 5.000:1, besonders bevorzugt von 1.000:1, umfasst. Selbst die beiden extremen Verhältnisse 10:1 und 10.000:1 erhöhen die Abbaugeschwindigkeit des Blutalkohols, das Verhältnis von Fructose zu Vitamin-B-Komplex-Komponenten von 1.000:1 hat sich jedoch als optimal für den beschleunigten Alkohol-Abbau im Körper erwiesen.

Vorzugsweise sind die Komponenten des Vitamin-B-Komplexes ausgewählt aus Vitamin B1, B2, B6, B12, Biotin, Niacin, Pantothensäure, Folsäure, Adenin, Cholin, Adenosinphosphate, Orotsäure, Pangamsäure, Carnitin, 4-Aminobenzoesäure, myo-Inosit, Liponsäure und/oder Amygdalin. Vitamin B1, auch als Thiamin bekannt, wird im Körper in Thiaminpyrophosphat umgewandelt, ein Coenzym bei einer Reihe von Reaktionen, bei denen C-C-Bindungen gespalten werden. Es kann unter anderem auch als Thiaminhydrochlorid zugesetzt werden. Vitamin B2, auch Riboflavin genannt, wird im Dünndarm resorbiert, in FMN (Flavinmononucleotid) umgewandelt und in der Leber in FAD (Flavinadenindinucleotid) übergeführt, die beide Coenzyme bei Redoxreaktionen, unter anderem bei der Alkoholdehydrogenase, sind. Vitamin B6, auch Pyridoxal, Pyridoxin und Pyridoxamin genannt, ist Bestandteil des Pyridoxal-5-phosphats, das ein Cofaktor beim Glykogen-Abbau und im Aminosäurenstoffwechsel, z.B. als Coenzym von Decarboxylasen, ist. Bevorzugt wird dieser Stoff in Form von Pyridoxinhydrochlorid der Getränk-Zusammensetzung beigemengt. Vitamin B12, auch Cyanocobalamin genannt, weist eine komplexe Struktur auf und ist Bestandteil von Cobalamin-Coenzymen, wobei z.B. Methylcobalamin und Cobalamid unter anderem bei Umlagerungen mit Wasserstoff-Wanderung eine Rolle spielen. Biotin, auch Vitamin B7 oder H genannt, wird kovalent an Carboxylasen gebunden, Niacin, ein Oberbegriff für Nicotinsäure und Nicotinamid, ist als Bestandteil des NAD und dessen Phosphats NADP einer der wichtigsten Wasserstoffüberträger in der Zelle und hat einen schützenden und aufbauenden Effekt auf den Körper. Pantothensäure, auch als Vitamin B3 bzw. B5 bezeichnet, hat eine Vorläuferfunktion für Coenzym A, das eine zentrale Stellung im Stoffwechsel einnimmt. Folsäure, oder Vitamin M, B9 bzw. Bc, ist Bestandteil des Coenzyms Tetrahydrofolat. Diese genannten Beispiele (um nur einige zu nennen) zeigen deutlich, was für eine wichtige Rolle Vitamin-B-Komplex-Komponenten im Stoffwechsel spielen.

Vorzugsweise umfasst die Erfrischungsgetränk-Zusammensetzung die Vitamin-B-Komplex-Komponenten zu folgenden Gewichtsteilen, bezogen auf 15.000-20.000 Gesamtgewichtsteile Trockensubstanz: Vitamin B1 zu 0,1-10, insbesondere 1, Vitamin B2 zu 0,1-10, insbesondere 1,5, Vitamin B6 zu 0,1-10, insbesondere 1,5, Biotin zu 0,01-1, insbesondere 0,1, Niacin zu 0,1-100, insbesondere 10-30, Pantothensäure zu 0,1-100, insbesondere 1-10, Vitamin B12 zu 0,0001-0,1, insbesondere 0,001-0,01, und/oder Folsäure zu 0,01-10, insbesondere 0,1. Es ist natürlich selbstverständlich, dass nur einige dieser Komponenten (oder nur eine) in der Zusammensetzung enthalten sein können, bzw. dass die Komponenten auch andere Verhältnisse in Bezug auf das Trockensubstanz-Gewicht aufweisen können. Die Möglichkeiten der Variationen sind vielfältig, wobei bei den meisten Variationen ein beschleunigter Alkohol-Abbau erzielt wird. Die hier genannten bevorzugten Mengen zeigten einen besonders raschen Abbau. Da die Vitamin-B-Komplex-Komponenten wasserlöslich sind, wird im Falle eines Überschusses der ungebrauchte Teil aus dem Körper geschieden. Demnach besteht, selbst bei hohen Konzentrationen dieser Komponenten in der erfindungsgemäßen Zusammensetzung, keine Gefahr von Hypervitäminosen.

Ein besonders günstiges Resultat wird erzielt, wenn die Zusammensetzung weiters Mineralstoffe, insbesondere Magnesium und/oder Kalium und/oder Zink, umfasst. Da Mineralstoffe für eine Reihe von Stoffwechselvorgängen im Körper von großer Bedeutung sind, ist der Zusatz dieser Stoffgruppe in die Zusammensetzung des Getränks wichtig, wobei mehrere verschiedene Mineralstoffe zugesetzt werden können. Im Einzelnen ist Zink ein Bestandteil der Alkohol-Dehydrogenase, Kalium und Magnesium spielen im Stoffwechsel eine große Rolle, unter anderem ist Magnesium an allen durch ATP katalysierten Enzym-Reaktionen beteiligt. Mineralstoffe können in Kombination mit weiteren Lebensmittelzusatzstoffen beigefügt werden, so z.B. als Magnesiumglycerophosphat, Kaliumcitrat (Säureregulator), Zinkgluconat (Fruchtsäure), Calciumpantothenat, um nur einige Beispiele zu nennen. Erfindungsgemäß konnte daher erstmals gezeigt werden, dass die Aktivierung der ADH (durch Zinkionen) und die Aktivierung des Kohlenhydratstoffwechsels (durch Magnesium) direkt zu einem beschleunigten Alkohol-Abbau führen. Dies war bislang in der Literatur nicht beschrieben öder vorgeschlagen worden.

Bevorzugt umfasst die Zusammensetzung Magnesium, Kalium und/oder Zink zu folgenden Gewichtsteilen, bezogen auf 15.000-20.000 Gesamtgewichtsteile Trockensubstanz: Magnesium zu 10-1.000, insbesondere 100, Kalium zu 10-1.000, insbesondere 100, Zink zu 0,1-100, insbesondere 1-10. Auch diese Mengenangaben verstehen sich wiederum nur als Richtlinien, da verschiedene Verhältnisse sehr gute Resultate ergeben. Die hier bevorzugten Mengen zeigten die besten Ergebnisse.

Weiters ist von Vorteil, wenn die Zusammensetzung Aminosäuren umfasst, insbesondere L-Glutamin und/oder L-Arginin. Aminosäuren spielen eine wichtige Rolle in den verschiedenen Stoffwechselprozessen des menschlichen Körpers, so dass der Zusatz von Aminosäuren sich generell positiv auf den Alkoholabbau des Körpers auswirkt. Im Speziellen wirken besonders L-Glutamin und L-Arginin Alkohol-Abbau fördernd, wobei sie bevorzugt zu folgenden Gewichtsteilen, bezogen auf 15.000-20.000 Gesamtgewichtsteile Trockensubstanz, zugesetzt werden: L-Arginin zu 20-2.000, insbesondere 200, L-Glutamin zu 10-1.000, insbesondere 100. Diese Mengen erzielten optimale Alkohol-Abbau-Ergebnisse.

Weiters ist es günstig, wenn die Zusammensetzung Coffein, insbesondere zu 0,1-100, besonders bevorzugt zu 10-50, Gewichtsteilen, bezogen auf 15.000-20.000 Gesamtgewichtsteile Trockensubstanz, umfasst. Coffein (Thein, Guaranin, Trimethylxanthin) wirkt erregend auf das ZNS, regt Herztätigkeit, Stoffwechsel und Atmung an, Blutdruck, Körpertemperatur und Blutumlaufgeschwindigkeit steigen, die Blutgefäße im Hirn erweitern sich, während sie sich in den Eingeweiden verengen. Dies hat eine Unterdrückung der Müdigkeit, vorübergehende Besserung der Arbeitsleistung und Hebung der Stimmung zur Folge und wirkt den Effekten des Alkohols entgegen.

Vorzugsweise umfasst die Erfrischungsgetränk-Zusammensetzung Anthocyane, insbesondere zu 0,1-100, besonders bevorzugt zu 10 Gewichtsteilen, bezogen auf 15.000-20.000 Gesamtgewichtsteile Trockensubstanz. Anthocyane werden in der Lebensmittelindustrie häufig als Farbstoffe eingesetzt, wobei - je nach pH oder Komplexbildung mit Metallen - eine rote, blaue bzw. violette Farbe erreicht werden kann.

Weiters ist besonders vorteilhaft, wenn -die Zusammensetzung Taurin zu 10-1.000, vorzugsweise zu 100, Gewichtsteile, bezogen auf 15.000-20.000 Gesamtgewichtsteile Trockensubstanz, umfasst. Wird Taurin in diesem Verhältnis zugesetzt, wird die optimale Wirkung von Taurin sowie ein optimaler synergistischer Effekt des Kombinationsproduktes erreicht, so dass ein äußerst effektiver und schneller Alkohol-Abbau im Körper erreicht wird, wenn diese Zusammensetzung eingenommen wird.

Für einen optimalen Alkohol-Abbau ist es günstig, wenn die Zusammensetzung, bezogen auf 100 Gew.% Trockensubstanz, jeweils 0,0001-0,1%, insbesondere 0,001-0,01%, Vitamin B1, Vitamin B2 und/oder Vitamin B6, 0,000001-0,001, insbesondere 0,00001-0,0001% Vitamin B12, jeweils 0,00001-0,01%, insbesondere 0,0001-0,001%, Biotin und/oder Folsäure, jeweils 0,001-1%, insbesondere 0,01-0,1%, Niacin, Pantothensäure, Zink und/oder Anthocyane, jeweils 0,01-10%, insbesondere 0,1-1%, Magnesium, Kalium, L-Glutamin, Coffein und/oder Taurin, 0,01-100%, insbesondere 0,1-10%, L-Arginin und/oder 50-99,99%, insbesondere 95-98%, Fructose umfasst. Wie oben erwähnt sind unterschiedliche Kombinationen wirksam zur Steigerung der Alkohol-Abbau-Geschwindigkeit, diese hier genannten Verhältnisse haben sich jedoch als optimal erwiesen.

Eine weitere günstige Verwendung ist durch den Zusatz weiterer Vitamine, insbesondere Vitamin C, zur Zusammensetzung gegeben. Dabei sind alle in der Lebensmittelindustrie üblichen Vitamine, in den üblichen Konzentrationen, möglich. Vitamin C ist zur Steigerung der Alkohol-Abbau-Geschwindigkeit nicht notwendig, kann jedoch, wie dies normalerweise bei Getränken, insbesondere Fruchtsäften, der Fall ist, aus gesundheitlichen Gründen (z.B. zur Unterstützung der Immunabwehr) zugesetzt werden.

Vorzugsweise umfasst die Zusammensetzung weitere Zusatzstoffe, insbesondere Aromastoffe, Konservierungsstoffe, Farbstoffe, Antioxidantien, Elektrolyte, Enzyme, Pflanzenextrakte, Glycerinphosphate, Säureregulatoren und/oder Säuerungsmittel, insbesondere Fruchtsäuren. Diese Stoffe haben im Allgemeinen keinen besonderen Einfluss auf die Alkohol-Abbau-Geschwindigkeit, sie werden lediglich, wie bei jedem Lebensmittel und bei Getränken, aus lebensmitteltechnischen Gründen zugesetzt. Als Säureregulatoren können auch antiazide Mittel zugesetzt werden, die der überhöhten Säuerung im Magen, insbesondere nach exzessivem Weingenuss, entgegenwirken.

Weitere zusätzliche Stoffe können auch Histamine sein, die allergischen Reaktionen, die bei manchen Personen gegen verschiedene Inhaltsstoffe von alkoholischen Getränken auftreten, entgegenwirken. Weiters können auch, zumindest in geringen Konzentrationen, Chinine zugesetzt werden, die sich auf den Alkoholabbau ebenfalls günstig und beschleunigend auswirken und auch muskelentspannend sind.

Eine besonders bevorzugte Verwendung wird dadurch erreicht, dass die Erfrischungsgetränk- Zusammensetzung weitere Zucker und/oder Süßstoffe umfasst. Zum Süßen der Zusammensetzung können sowohl künstliche als auch natürliche Süßstoffe beigefügt werden. Neben Fructose kann weiters jeder Zucker zugesetzt werden, wie etwa Glucose, Galactose, Lactose, etc.. Dadurch erhält das Getränk, neben dem Zusatz von Fructose, einen noch süßeren Geschmack. Neben der Süßungswirkung wirkt Lactose ebenfalls Alkohol abbauend, so dass der Gehalt an Alkohol durch den Zusatz von Lactose weiter verringert wird.

Die erfindungsgemäße Verwendung der eingangs angeführten Art ist weiters vorzugsweise dadurch gekennzeichnet, dass das Erfrischungsgetränk zusätzlich zu der oben beschriebenen erfindungsgemäßen Zusammensetzung weiters ein flüssiges Lebensmittel umfasst. Durch Auflösen/Suspendieren der erfindungsgemäßen Zusammensetzung mit dem flüssigen Lebensmittel wird ein flüssiges Getränk zum Abbau des Blutalkoholspiegels erhalten. Die Flüssigkeit ist in erster Linie Wasser, wobei sowohl kohlensäurehältiges als auch -freies Wasser verwendet werden kann. Es kann aber auch jede andere körperverträgliche Flüssigkeit zugesetzt werden, wie etwa Fruchtsäfte, Milch, Tee, Kaffee und Ähnliches. Weiters ist es auch möglich, ein alkoholhältiges Getränk, z.B. einen alkoholhältigen Cocktail, zuzusetzen, so dass die Alkohol abbauende Wirkung gleichzeitig mit dem Alkoholgenuss eintritt.

Weiters wird eine erfindungsgemäße Verwendung einer Zusammensetzung zur Verfügung gestellt, die als Sirup oder aber auch als Trockensubstanz vorliegt. Der Sirup bzw. die Trockensubstanz können mit jeder körperverträglichen Flüssigkeit verdünnt bzw. aufgelöst werden, um ein möglichst gut schmeckendes Getränk zu erhalten. Die Trockensubstanz ist z.B. pulver- oder tablettenförmig herstellbar, so dass sie leicht handhabbar und in Portionen abfüllbar ist und leicht überallhin mitgenommen und - diskret - verwendet werden kann. Besonders leicht handhabbar sind weiters Brausetabletten oder Brausepulver, die in der Flüssigkeit leicht lösbar sind und durch die Kohlensäure angenehm prickelnd schmecken.

Es ist selbstverständlich, dass sich die Erfindung auf jede weitere mögliche Konsistenz bezieht, wobei die Alkohol abbauende Zusammensetzung enthaltend zumindest Fructose und Vitamin-B-Komplex-Komponenten natürlich sowohl durch Trinken, als auch durch Kauen, Lutschen, Schlucken, um nur einige Beispiele zu nennen, aufgenommen werden kann.

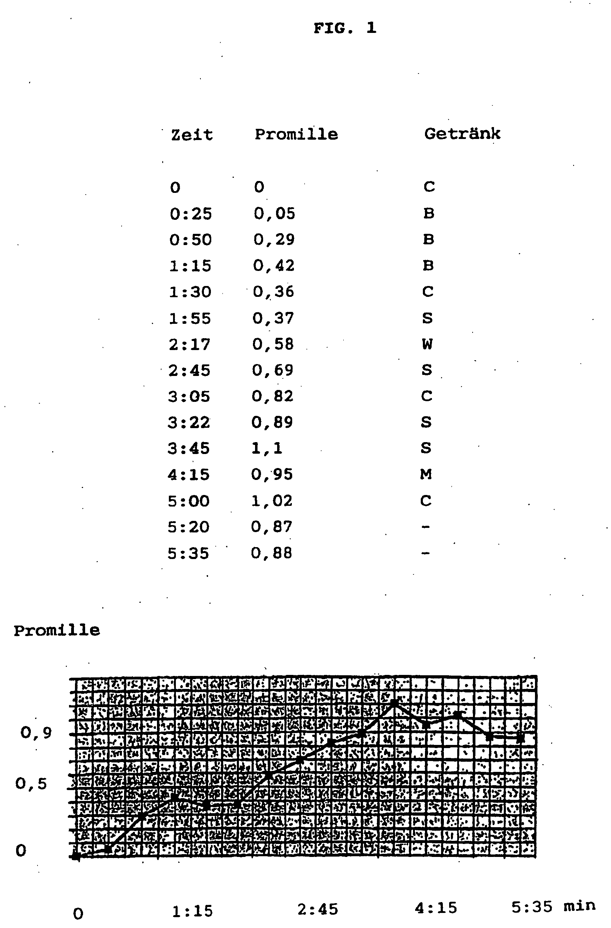

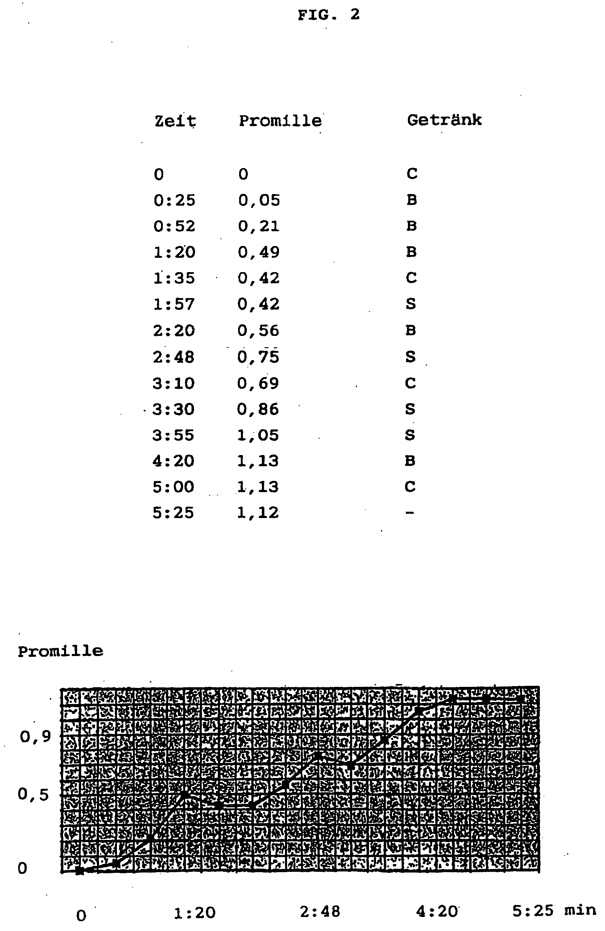

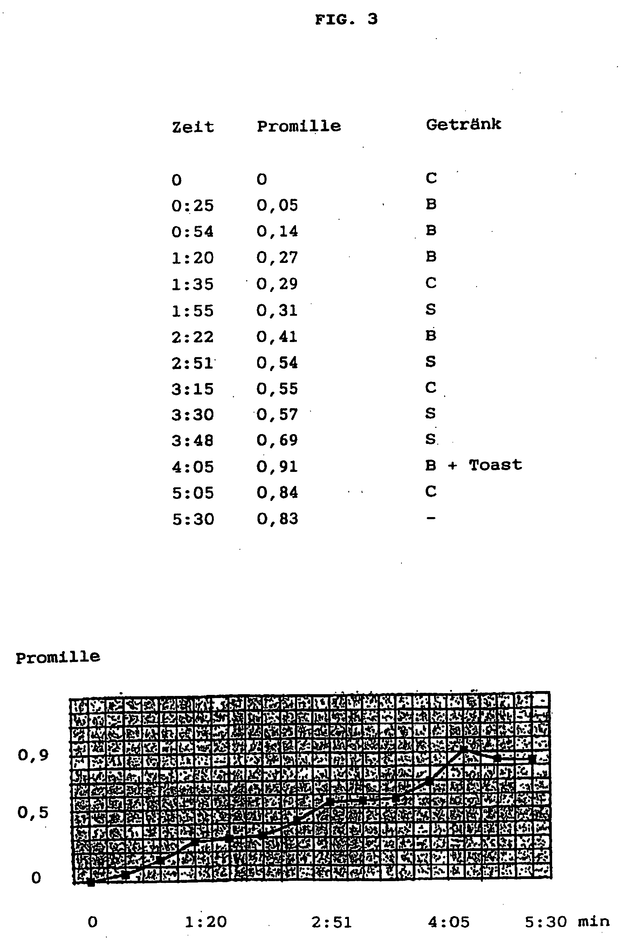

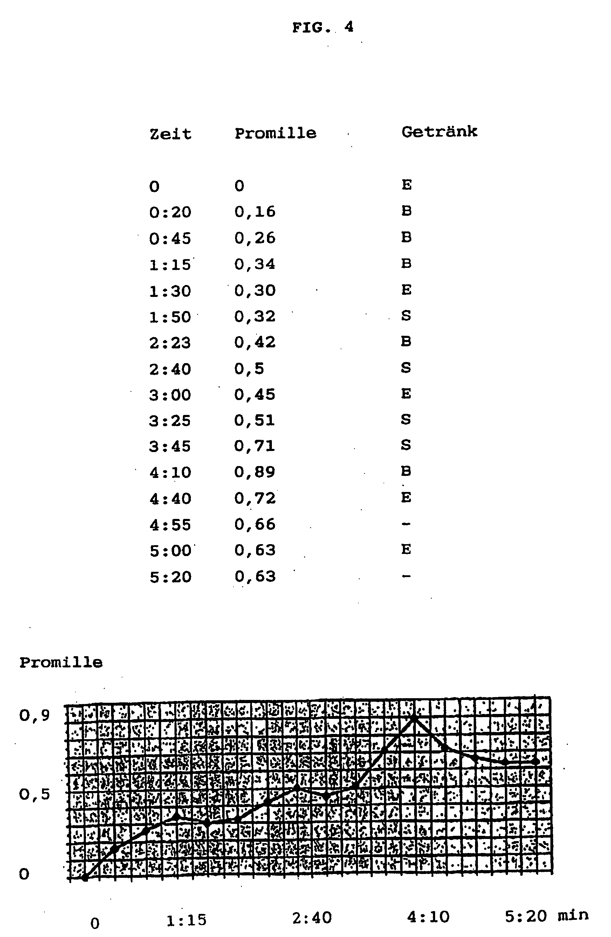

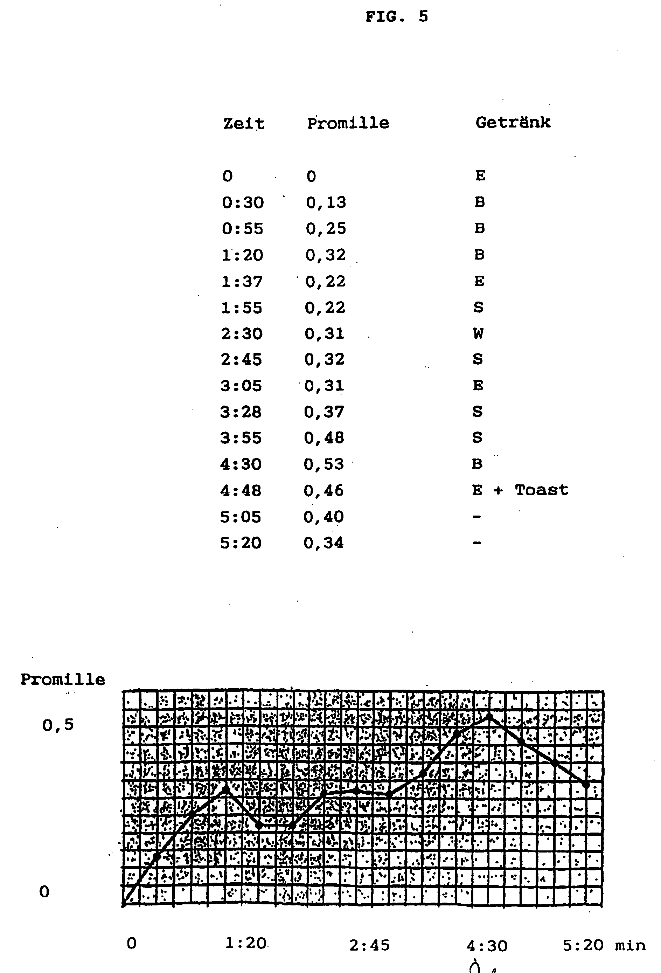

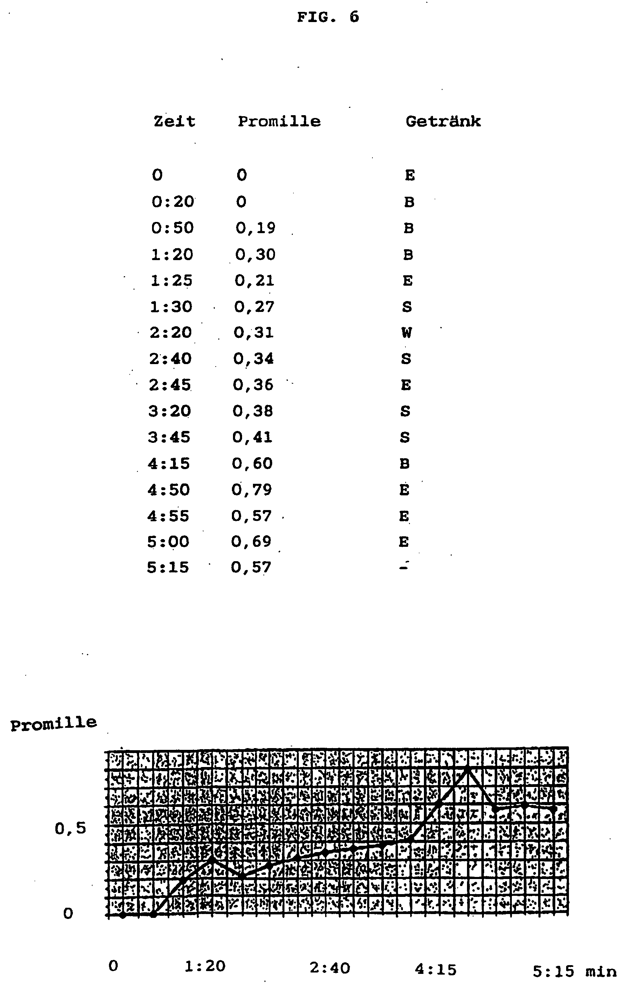

Die Erfindung wird anhand des nachfolgenden Beispiels und der Zeichnungsfiguren, auf die sie selbstverständlich nicht eingeschränkt ist, weiter erläutert werden. Im Einzelnen zeigen in der Zeichnung: die Figuren 1-3 jeweils einen Verum-Versuch und die Figuren 4-6 jeweils einen Placebo-Versuch, wobei der Promille-Gehalt an Alkohol in der Zeit (min) als Kurve dargestellt ist und in der Tabelle die einzelnen Ergebnisse aufgelistet sind (E=erfindungsgemäßes Getränk, S=Schnaps, B=Bier, W=Wein, C=Coca Cola, M=Mineral, -=kein Getränk). Folgende Versuchspersonen wurden für den Test herangezogen: Fig.1: weiblich, 1975 geboren, 160 cm, 55 kg; Fig.2: männlich, 1977 geboren, 175 cm, 68 kg; Fig.3: männlich, 1968 geboren, 174 cm, 72 kg; Fig.4: männlich, 1963 geboren, 178 cm, 85 kg; Fig.5: männlich, 1957 geboren, 185 cm, 106 kg; Fig.6: männlich, 1950 geboren, 180 cm, 82 kg. Bei den 3 Verum- Versuchen (Fig.1-3) ist eindeutig eine gesteigerte Alkohol-Abbau-Geschwindigkeit im Vergleich zu den Placebo-Versuchen (Fig.4-6) erkennbar.

### Beispiel :

Folgende Stoffe wurden in 250 ml Wasser zugesetzt: 15 mg Niacin, 5 mg Pantothensäure, 1,6 mg Vitamin B6, 1,5 mg Vitamin B2, 1,1 mg Vitamin B1, 0,1 mg Folsäure, 0,1 mg Biotin, 2 mg Vitamin B12, 200 mg L-Arginin, 100 mg L-Glutamin, 100 mg Taurin, 100 mg Mg, 100 mg K, 5 mg Zn, 10 mg Anthocyane, 30 mg Coffein, 17.500 mg Fructose.

Einem Teilnehmerkreis von 10 Personen wurde über einen Zeitraum von 2,5 Stunden eine jeweils gleiche Menge und Sorte von Alkohol (Bier und Schnaps) verabreicht. 5 Personen erhielten zu den Zeitpunkten 0, 60, 120 und 180 Minuten jeweils 250 ml des erfindungsgemäßen Getränks (Verum-Gruppe), 5 Personen jeweils 250 ml eines Coca Cola-Getränks (Kontrollgruppe). Die Alkohol-Messwerte wurden im Abstand von 15 Minuten über einen Zeitraum von 4 Stunden erhoben. Der folgende Tag wurde unter völliger Alkoholabstinenz verbracht. Am darauffolgenden Tag wurde der Vorversuch wiederholt, diesmal jedoch erhielt die Kontrollgruppe das erfindungsgemäße Getränk, die Verum-Gruppe das Coca Cola-Getränk.

Die Versuchspersonen waren erwachsene Personen beiderlei Geschlechts in gutem Allgemeinzustand. Personen, die unter medikamentöser Therapie standen, sowie Personen mit Leber- und Nieren-Funktionsstörungen wurden als Versuchsteilnehmer ausgeschlossen. Die Alkohol-Messwerte wurden mit dem in Österreich zugelassenen Alkohol-Messgerät "Promillomat" aus der Atemluft bestimmt.

Ergebnisse: Die durchschnittliche Alkohol-Abbaugeschwindigkeit betrug bei der Kontrollgruppe 0,13% pro Stunde, in der Verum-Gruppe dagegen 0,19% pro Stunde. Dies entspricht einer durchschnittlichen Steigerung der Alkohol-Abbaugeschwindigkeit unter dem erfindungsgemäßen Getränk von 46%. Die errechneten Werte sind arithmetische Mittelwerte.

## Patentansprüche

1. Verwendung einer Erfrischungsgetränk-Zusammensetzung zur Steigerung der Alkohol-Abbau-Kapazität des Körpers enthaltend Fructose, **dadurch gekennzeichnet, dass** die Zusammensetzung weiters eine oder mehrere Komponenten des Vitamin-B-Komplexes sowie Taurin umfasst.

2. Verwendung nach Anspruch 1, **dadurch gekennzeichnet, dass** die Zusammensetzung Fructose und Vitamin-B-Komplex-Komponenten in einem Gewichtsverhältnis von 10:1 bis 10.000:1, insbesondere von 100:1 bis 5.000:1, besonders bevorzugt von 1.000:1, umfasst.

3. Verwendung nach Anspruch 1 oder 2, **dadurch gekennzeichnet, dass** die Komponenten des Vitamin-B-Komplexes ausgewählt sind aus Vitamin B1, B2, B6, B12, Biotin, Niacin, Pantothensäure, Folsäure, Adenin, Cholin, Adenosinphosphate, Orotsäure, Pangamsäure, Carnitin, 4-Aminobenzoesäure, myo-Inosit, Liponsäure und/oder Amygdalin.

4. Verwendung nach einem der Ansprüche 1 bis 3, **dadurch gekennzeichnet, dass** die Zusammensetzung die Vitamin-B-Komplex-Komponenten zu folgenden Gewichtsteilen, bezogen auf 15.000-20.000 Gesamtgewichtsteile Trockensubstanz, umfasst: Vitamin B1 zu 0,1-10, insbesondere 1, Vitamin B2 zu 0,1-10, insbesondere 1,5, Vitamin B6 zu 0,1-10, insbesondere 1,5, Biotin zu 0,01-1, insbesondere 0,1, Niacin zu 0,1-100, insbesondere 10-30, Pantothensäure zu 0,1-100, insbesondere 1-10, Vitamin B12 zu 0,0001-0,1, insbesondere 0,001-0,01, und/oder Folsäure zu 0,01-10, insbesondere 0,1.

5. Verwendung nach einem der Ansprüche 1 bis 4, **dadurch gekennzeichnet, dass** die Zusammensetzung weiters Mineralstoffe, insbesondere Magnesium und/oder Kalium und/oder Zink, umfasst.

6. Verwendung nach Anspruch 5, **dadurch gekennzeichnet, dass** die Zusammensetzung Magnesium, Kalium und/oder Zink zu folgenden Gewichtsteilen, bezogen auf 15.000-20.000 Gesamtgewichtsteile Trockensubstanz, umfasst: Magnesium zu 10-1.000, insbesondere 100, Kalium zu 10-1.000, insbesondere 100, Zink zu 0,1-100, insbesondere 1-10.

7. Verwendung nach einem der Ansprüche 1 bis 6, **dadurch gekennzeichnet, dass** die Zusammensetzung Aminosäuren umfasst, insbesondere L-Glutamin und/oder L-Arginin.

8. Verwendung nach Anspruch 7, **dadurch gekennzeichnet, dass** die Zusammensetzung L-Arginin und/oder L-Glutamin zu folgenden Gewichtsteilen, bezogen auf 15.000-20.000 Gesamtgewichtsteile Trockensubstanz umfasst: L-Arginin zu 20-2.000, insbesondere 200, L-Glutamin zu 10-1.000, insbesondere 100.

9. Verwendung nach einem der Ansprüche 1 bis 8, **dadurch gekennzeichnet, dass** die Zusammensetzung Coffein, insbesondere zu 0,1-100, besonders bevorzugt zu 10-50, Gewichtsteilen, bezogen auf 15.000-20.000 Gesamtgewichtsteile Trockensubstanz, umfasst.

10. Verwendung nach einem der Ansprüche 1 bis 9, **dadurch gekennzeichnet, dass** die Zusammensetzung Anthocyane, insbesondere zu 0,1-100, besonders bevorzugt zu 10 Gewichtsteilen, bezogen auf 15.000-20.000 Gesamtgewichtsteile Trockensubstanz, umfasst.

11. Verwendung nach einem der Ansprüche 1 bis 10, **dadurch gekennzeichnet, dass** die Zusammensetzung Taurin, zu 10-1.000, vorzugsweise zu 100, Gewichtsteile, bezogen auf 15.000-20.000 Gesamtgewichtsteile Trockensubstanz, umfasst.

12. Verwendung nach einem der Ansprüche 1 bis 11, **dadurch gekennzeichnet, dass** die Zusammensetzung, bezogen auf 100 Gew.% Trockensubstanz jeweils 0,0001-0,1%, insbesondere 0,001-0,01%, Vitamin B1, Vitamin B2 und/oder Vitamin B6, 0,000001-0,001, insbesondere 0,00001-0,0001 Vitamin B12, jeweils 0.00001-0,01%, insbesondere 0,0001-0,001%, Biotin und/oder Folsäure, jeweils 0,001-1%, insbesondere 0,01-0,1%, Niacin, Pantothensäure, Zink und/oder Anthocyane, jeweils 0,01-10%, insbesondere 0,1-1%, Magnesium, Kalium, L-Glutamin, Coffein und/oder Taurin, 0,01-100%, insbesondere 0,1-10%, L-Arginin und/oder 50-99,99%, insbesondere 95-98%, Fructose umfasst.

13. Verwendung nach einem der Ansprüche 1 bis 12, **dadurch gekennzeichnet, dass** die Zusammensetzung weitere Vitamine, insbesondere Vitamin C, umfasst.

14. Verwendung nach einem der Ansprüche 1 bis 13, **dadurch gekennzeichnet, dass** die Zusammensetzung weitere Zusatzstoffe umfasst, insbesondere Aromastoffe, Konservierungsstoffe, Farbstoffe, Antioxidantien, Elektrolyte, Enzyme, Pflanzenextrakte, Giycerinphosphate, Säureregulatoren und/oder Säuerungsmittel, insbesondere Fruchtsäuren.

15. Verwendung nach einem der Ansprüche 1 bis 14, **dadurch gekennzeichnet, dass** die Zusammensetzung weitere Zucker und/oder Süßstoffe umfasst.

16. Verwendung eines Erfrischungsgetränks zur Steigerung der Alkohol-Abbau-Kapazität des Körpers, **dadurch gekennzeichnet, dass** es zusätzlich zu der Zusammensetzung wie in einem der Ansprüche 1 bis 15 definiert weiters ein flüssiges Lebensmittel umfasst.

17. Verwendung nach einem der Ansprüche 1 bis 15, **dadurch gekennzeichnet, dass** die Zusammensetzung als Sirup vorliegt.

18. Verwendung nach einem der Ansprüche 1 bis 15, **dadurch gekennzeichnet, dass** die Zusammensetzung als Trockensubstanz vorliegt.

19. Verwendung eines Sirups zur Steigerung der Alkohol-Abbau-Kapazität des Körpers, **dadurch gekennzeichnet, dass** er eine Zusammensetzung wie in einem der Ansprüche 1 bis 15 definiert aufweist.

20. Verwendung einer Trockensubstanz zur Steigerung der Alkohol-Abbau-Kapazität des Körpers, **dadurch gekennzeichnet, dass** sie eine Zusammensetzung wie in einem der Ansprüche 1 bis 15 definiert aufweist.

## Claims

1. The use of a refreshing drink composition containing fructose for increasing the alcohol degradation capacity of the body, **characterised in that** the composition further comprises one or more components of the vitamin B complex as well as taurine.

2. The use according to claim 1, **characterised in that** the composition comprises fructose and vitamin B complex components in a weight ratio of from 10:1 to 10,000:1, in particular from 100:1 to 5,000:1, particularly preferred from 1,000:1.

3. The use according to claim 1 or 2, **characterised in that** the components of the vitamin B complex are selected from vitamin B1, B2, B6, B12, biotin, niacin, pantothenic acid, folic acid, adenine, choline, adenosine phosphate, orotic acid, pangamic acid, carnitine, 4-aminobenzoic acid, myo-inositol, liponic acid and/or amygdaline.

4. The use according to any one of claims 1 to 3, **characterised in that** the composition comprises the vitamin B complex components in the following parts by weight, based on a total of 15,000-20,000 parts by weight of the dry substance: vitamin B1, 0.1-10, in particular 1; vitamin B2, 0.1-10, in particular 1.5; vitamin B6, 0.1-10, in particular 1.5, biotin, 0.01-1, in particular 0.1; niacin 0.1-100, in particular 10-30; pantothenic acid, 0.1-100, in particular 1-10; vitamin B12, 0.0001-0.1, in particular 0.001-0.01; and/or folic acid, 0.01-10, in particular 0.1.

5. The use according to any one of claims 1 to 4, **characterised in that** the composition further comprises mineral substances, in particular magnesium and/or potassium and/or zinc.

6. The use according to claim 5, **characterised in that** the composition comprises magnesium, potassium and/or zinc in the following parts by weight, based on a total of 15,000-20,000 parts by weight of dry substance: magnesium, 10-1,000, in particular 100; potassium: 10-1,000, in particular 100; zinc, 0.1-100, in particular 1-10.

7. The use according to any one of claims 1 to 6, **characterised in that** the composition comprises amino acids, in particular L-glutamine and/or L-arginine.

8. The use according to claim 7, **characterised in that** the composition comprises L-arginine and/or L-glutamine in the following parts by weight, based on a total of 15,000-20,000 parts by weight of dry substance: L-arginine, 20-2,000, in particular 200; L-glutamine, 10-1,000, in particular 100.

9. The use according to any one of claims 1 to 8, **characterised in that** the composition comprises caffeine, in particular 0.1-100, particularly preferably 10-50 parts by weight, based on a total of 15,000-20,000 parts by weight of dry substance.

10. The use according to any one of claims 1 to 9, **characterised in that** the composition comprises anthocyans, in particular 0.1-100, particularly preferably 10 parts by weight, based on a total of 15,000-20,000 parts by weight of dry substance.

11. The use according to any one of claims 1 to 10, **characterised in that** the composition comprises 10-1,000, preferably 100, parts by weight of taurine, based on a total of 15,000-20,000 parts by weight of dry substance.

12. The use according to any one of claims 1 to 11, **characterised in that**, based on 100 parts by weight of dry substance, the composition comprises 0.0001-0.1%, in particular 0.001-0.01%, each of vitamin B1, vitamin B2 and/or vitamin B6, 0.000001-0.001, in particular 0.00001-0.0001% of vitamin B12, 0.00001-0.01%, in particular 0.0001-0.001%, each of biotin and/or folic acid, 0.001-1%, in particular 0.01-0.1%, each of niacin, pantothenic acid, zinc and/or anthocyans, 0.01-10%, in particular 0.1-1%, each of magnesium, potassium, L-glutamine, caffeine and/or taurine, 0.01-100%, in particular 0.1-10%, of L-arginine, and/or 50-99.99%, in particular 95-98% of fructose.

13. The use according to any one of claims 1 to 12, **characterised in that** the composition comprises further vitamins, in particular vitamin C.

14. The use according to any one of claims 1 to 13, **characterised in that** the composition comprises further additives, in particular flavoring agents, preserving agents, coloring agents, antioxidants, electrolytes, enzymes, plant extracts, glycerolphosphates, acid regulators and/or acidifiers, in particular fruit acids.

15. The use according to any one of claims 1 to 14, **characterised in that** the composition comprises further sugars and/or sweeteners.

16. The use of a refreshing drink for increasing the alcohol degradation capacity of the body, **characterised in that** in addition to the composition as defined in any one of claims 1 to 15 it further comprises a liquid foodstuff.

17. The use according to any one of claims 1 to 15, **characterised in that** the composition is provided as a syrup.

18. The use according to any one of claims 1 to 15, **characterised in that** the composition is provided as a dry substance.

19. The use of a syrup for increasing the alcohol degradation capacity of the body, **characterised in that** it comprises a composition as defined in any one of claims 1 to 15.

20. The use of a dry substance for increasing the alcohol degradation capacity of the body, **characterised in that** it comprises a composition as defined in any one of claims 1 to 15.

## Revendications

1. Utilisation d'une composition de boisson rafraîchissante pour augmenter la capacité de dégradation de l'alcool dans le corps, contenant du fructose, **caractérisée en ce que** la composition comprend en outre un ou plusieurs composants du complexe de vitamine B ainsi que de la taurine.

2. Utilisation selon la revendication 1, **caractérisée en ce que** la composition comprend du fructose et des composants du complexe de vitamine B dans un rapport pondéral de 10:1 à 10 000:1, en particulier de 100:1 à 5000:1, tout particulièrement de 1000:1.

3. Utilisation selon la revendication 1 ou 2, **caractérisée en ce que** les composants du complexe de vitamine B sont choisis parmi les vitamines B1, B2, B6, B12, la biotine, la niacine, l'acide pantothénique, l'acide folique, l'adénine, la choline, le phosphate d'adénosine, l'acide orotique, l'acide pangamique, la carnitine, l'acide 4-aminobenzoïque, la myo-inosite, l'acide liponique et/ou l'amygdaline.

4. Utilisation selon l'une quelconque des revendications 1 à 3, **caractérisée en ce que** la composition comprend les composants du complexe de vitamine B à raison des parties en poids suivantes, par rapport à 15 000 à 20 000 parties en poids au total de matière sèche : pour la vitamine B1 0,1 à 10, en particulier 1, pour la vitamine B2 0,1 à 10, en particulier 1,5, pour la vitamine B6 0,1 à 10, en particulier 1,5, pour la biotine 0,01 à 1, en particulier 0,1, pour la niacine 0,1 à 100, en particulier 10 à 30, pour l'acide pantothénique 0,1 à 100, en particulier 1 à 10, pour la vitamine B12 0,0001 à 0,1, en particulier 0,001 à 0,01, et/ou pour l'acide folique 0,01 à 10, en particulier 0,1.

5. Utilisation selon l'une quelconque des revendications 1 à 4, **caractérisée en ce que** la composition comprend par ailleurs des substances minérales, en particulier du magnésium et/ou du potassium et/ou du zinc.

6. Utilisation selon la revendication 5, **caractérisée en ce que** la composition comprend du magnésium, du potassium et/ou du zinc en parties en poids suivantes, par rapport à 15 000 à 20 000 parties en poids au total de matière sèche : pour le magnésium 10 à 1000, en particulier 100, pour le potassium 10 à 1000, en particulier 100, pour le zinc 0,1 à 100, en particulier 1 à 10.

7. Utilisation selon l'une quelconque des revendications 1 à 6, **caractérisée en ce que** la composition comprend des acides aminés, en particulier de la L-glutamine et/ou de la L-arginine.

8. Utilisation selon la revendication 7, **caractérisée en ce que** la composition comprend de la L-arginine et/ou de la L-glutamine en parties en poids suivantes, par rapport à 15 000 à 20 000 parties en poids au total de matière sèche : pour la L-arginine 20 à 2000, en particulier 200, et pour la L-glutamine 10 à 1000, en particulier 100.

9. Utilisation selon l'une quelconque des revendications 1 à 8, **caractérisée en ce que** la composition comprend de la caféine, en particulier 0,1 à 100, mieux encore 10 à 50, parties en poids par rapport à 15 000 à 20 000 parties en poids au total de matière sèche.

10. Utilisation selon l'une quelconque des revendications 1 à 9, **caractérisée en ce que** la composition comprend des anthocyanes, en particulier 0,1 à 100, mieux encore 10, parties en poids par rapport à 15 000 à 20 000 parties en poids au total de matière sèche.

11. Utilisation selon l'une quelconque des revendications 1 à 10, **caractérisée en ce que** la composition comprend de la taurine à raison de 10 à 1000, de préférence 100, parties en poids par rapport à 15 000 à 20 000 parties en poids au total de matière sèche.

12. Utilisation selon l'une quelconque des revendications 1 à 11, **caractérisée en ce que** la composition comprend, par rapport à 100% en poids de matière sèche, respectivement 0,0001 à 0,1%, en particulier 0,001 à 0,01%, de vitamine B1, de vitamine B2 et/ou de vitamine B6, 0,000001 à 0,001%, en particulier 0,00001 à 0,000 1 % de vitamine B12, respectivement 0,00001 à 0,0 1 %, en particulier 0,0001 à 0,001%, de biotine et/ou d'acide folique, respectivement 0,001 à 1%, en particulier 0,01 à 0,1%, de niacine, d'acide pantothénique, de zinc et/ou d'anthocyanes, respectivement 0,01 à 10%, en particulier 0,1 à 1%, de magnésium, de potassium, de L-glutamine, de caféine et/ou de taurine, 0,01 à 100%, en particulier 0,1 à 10%, de L-arginine et/ou 50 à 99,99%, en particulier 95 à 98%, de fructose.

13. Utilisation selon l'une quelconque des revendications 1 à 12, **caractérisée en ce que** la composition comprend d'autres vitamines, en particulier de la vitamine C.

14. Utilisation selon l'une quelconque des revendications 1 à 13, **caractérisée en ce que** la composition comprend d'autres additifs, en particulier des substances aromatiques, des conservateurs, des colorants, des antioxydants, des électrolytes, des enzymes, des extraits végétaux, du phosphate de glycérine, des régulateurs d'acidité et/ou des agents d'acidification, en particulier des acides de fruits.

15. Utilisation selon l'une quelconque des revendications 1 à 14, **caractérisée en ce que** la composition comprend d'autres sucres et/ou d'autres édulcorants.

16. Utilisation d'une boisson de rafraîchissement pour augmenter la capacité de dégradation de l'alcool dans le corps, **caractérisée en ce qu'**elle comprend en plus de la composition telle que définie dans l'une quelconque des revendications 1 à 15, en outre un aliment liquide.

17. Utilisation selon l'une quelconque des revendications 1 à 15, **caractérisée en ce que** la composition se présente sous la forme d'un sirop.

18. Utilisation selon l'une quelconque des revendications 1 à 15, **caractérisée en ce que** la composition se présente sous la forme d'une matière sèche.

19. Utilisation d'un sirop pour augmenter la capacité de dégradation de l'alcool dans le corps, **caractérisée en ce qu'**il présente une composition telle que définie dans l'une quelconque des revendications 1 à 15.

20. Utilisation d'une matière sèche pour augmenter la capacité de dégradation de l'alcool dans le corps, **caractérisée en ce qu'**elle présente une composition telle que définie dans l'une quelconque des revendications 1 à 15.
